# EUROPEAN PATENT SPECIFICATION

(11) **EP 1 685 873 B1**
(45) Date of publication and mention of the grant of the patent: **19.03.2008**
(21) Application number: 06250529.2
(22) Date of filing: 31.01.2006
(51) Int. Cl.: A61M 39/02, A61F 5/00, A61L 31/14

(54) **A surgical implantable injection port having an absorbable fastener**
Chirurgisch implantierbarer Injektionszugang mit absorbierbaren Befestigungsmitteln
Orifice d'injection chirurgical implantable avec fixateur absorbable

(30) Priority: 01.02.2005 US 48155
(43) Date of publication of application: 02.08.2006
(73) Proprietor: ETHICON ENDO-SURGERY, INC., Cincinnati, Ohio 45242 (US)
(72) Inventor: Conlon, Sean P., Loveland, Ohio 45140 (US)
(74) Representative: Mercer, Christopher Paul

(56) References cited:
- EP-A- 0 853 949
- EP-A- 1 488 824
- DE-A1- 19 751 791
- US-A- 5 324 307
- US-A- 5 848 989

## Description

### Field of the Invention

The present invention has application in conventional endoscopic and open surgical instrumentation as well as application in robotic-assisted surgery. The present invention has even further relation to adjustable surgically implantable bands, such as gastric bands for the treatment of obesity.

### Background of the Invention

The percentage of the world's population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity.

Most surgical procedures for treatment of morbid obesity may generally be classified as either being directed toward the prevention of absorption of food (malabsorption), or restriction of stomach to make the patient feel full (gastric restriction) The most common malabsorption and gastric restriction technique is the gastric bypass. In variations of this technique, the stomach is horizontally divided into two isolated pouches, with the upper pouch having a small food capacity. The upper pouch is connected to the small intestine, or jejunum, through a small stoma, which restricts the processing of food by the greatly reduced useable stomach. Since food bypass much of the intestines, the amount of absorption of food is greatly reduced.

There are many disadvantages to the above procedure. Typically the above mentioned procedure is performed in an open surgical environment. Current minimally invasive techniques are difficult for surgeons to master, and have many additional drawbacks. Also, there is a high level of patient uneasiness with the idea of such a drastic procedure which is not easily reversible. In addition, all malabsorption techniques carry ongoing risks and side effects to the patient, including malnutrition and dumping syndrome.

Consequently, many patients and physicians prefer to undergo a gastric restriction procedure for the treatment of morbid obesity. One of the most common procedures involves the implantation of an adjustable gastric band. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent. In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patients new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding. Gastric bands typically include a flexible substantially non-extensible portion having an expandable, inflatable portion attached thereto. The inflatable portion is in fluid communication with a remote injection site, or port. Injection or removal of an inflation fluid into or from the interior of the inflatable portion is used to adjust the size of the stoma either during or following implantation. By enlarging the stoma, the patient can eat more food without feeling as full, but will not lose weight as fast. By reducing the size of the stoma, the opposite happens. Physicians regularly adjust the size of stoma to adjust the rate of weight loss.

For most fluid injection ports for the above described bands are attached underneath the skin to the fascia of a patient. Such ports are often provided with suture holes and the port is sutured to the tissue. However, alternative means of attaching the port to the patient, such as using integral hooks, can be used as well. Such other means for attaching the port to a patient are described in commonly assigned and copending U.S. Patent Application Serial Numbers: 10/741,785 filed December 19, 2003; EP-A-1 488 824 and EP-A-1 547 643. EP-A-1 488 824 forms the basis for the preamble of claim 1 appended hereto.

However, many of the prior art fasteners could cause patient discomfort, including pain. It is well known that once the port is placed a fibrotic capsule begins to grow over the port until it is completely enclapsuled. The rate at which the fibrotic capsule grows varies from patient to patient, but generally surgeons agree that the port is fully encapsulated after 2 months. Once the port has been captured by the fibrotic capsule, there is no longer a need for the port to be fastened with sutures or other types of fasteners. In fact, it would be desirable if these additional fastening means were no longer part of the port system so as to not cause patient discomfort.

### Summary of the Invention

In accordance with the present invention as defined in claim 1, there is provided an implantable surgical injection port having an undeployed position, and a deployed position wherein it is attached to tissue. The port includes a housing having a closed distal end, a open proximal end and a fluid reservoir therebetween. The port further includes a needle penetrable septum attached to the housing about the proximal end open. The port even further includes at least one attachment mechanism mounted to the housing for initially attaching the port to tissue wherein the attachment mechanism is made from a bioabsorbable material.

### Detailed Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
Figure 1 is a perspective view of a surgically implantable fluid port made in accordance with the present invention, showing the port attached to an adjustable gastric band;
Figure 2 is a perspective view of a surgically implantable fluid port made in accordance with the present invention;
Figure 3 is a cross section of the port shown in Figures 1 and 2, taken along line 3-3 in Figure 1; and
Figure 4 is a view similar to that of Figure 3 but showing the fluid port implanted within a patient.

### Detailed Description of the Invention

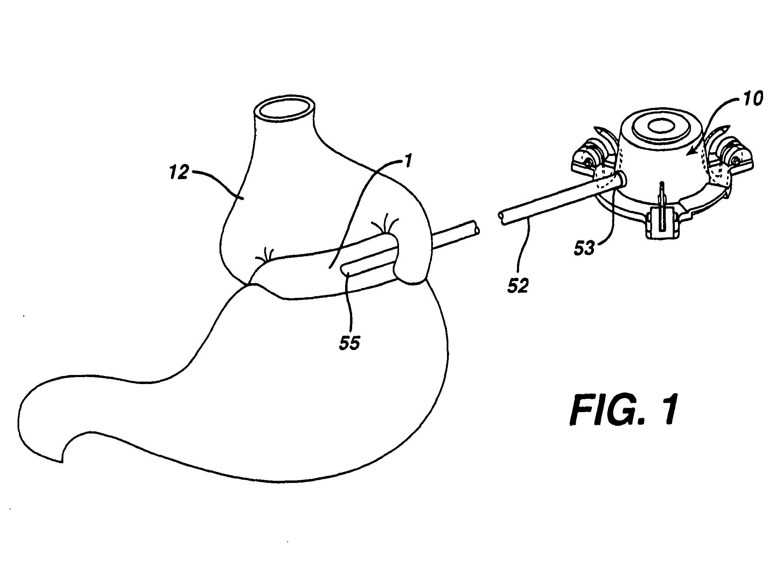

Referring now to the drawings wherein like numerals indicate the same elements throughout the views, as stated above there is shown in Figure 1 an adjustable gastric band 1 of the type described in the above mentioned incorporated references. Band 1 is implanted within a body of a patient to surround the stomach 12. The inflatable portion of the band is in fluid communication with injection port 10 via a catheter tube 52. Tube 52 has a proximal end 53 attached to the port 10 and a distal end 55 attached to adjustable gastric band 1. Port 10 can be used for a wide range of devices in the medical field and not only for gastric bands. For example the port can also used for vascular access for drug delivery.

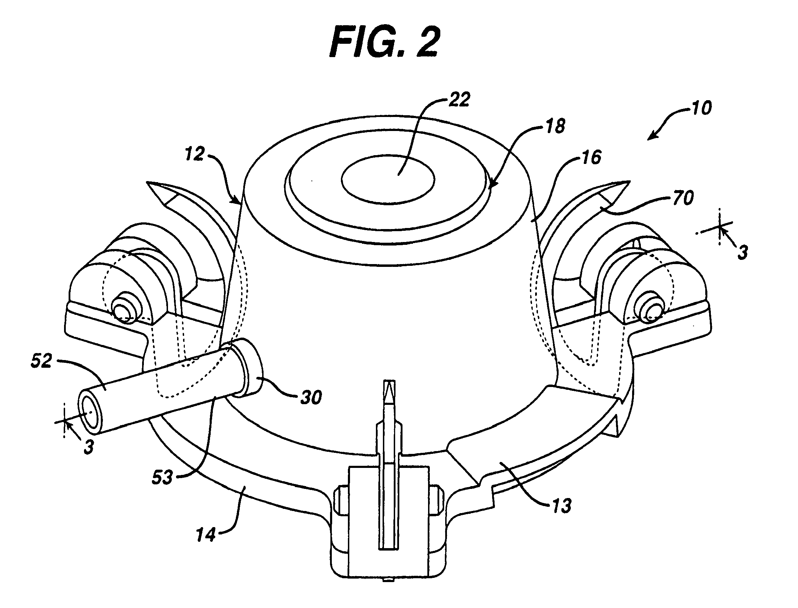

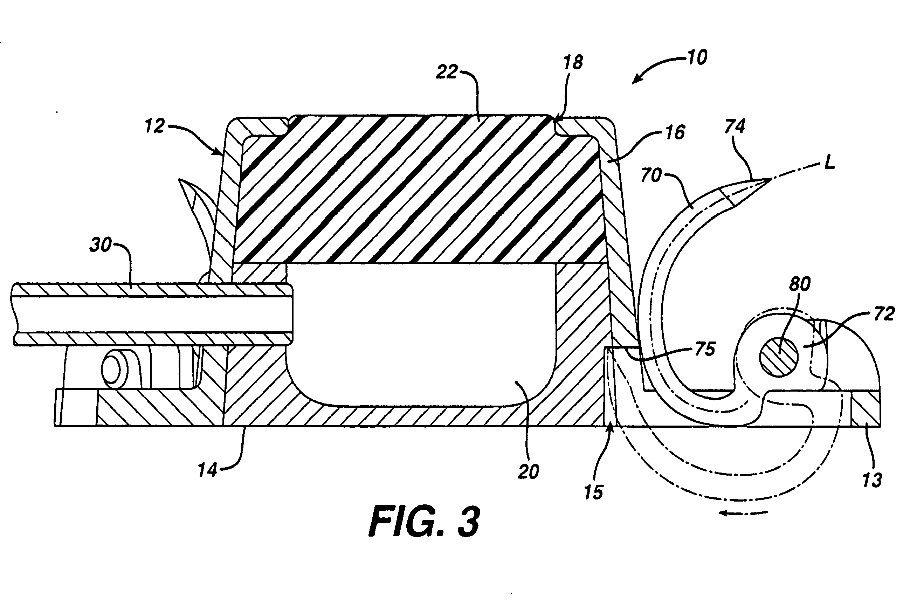

As seen from Figures 2 and 3, surgically implantable injection port 10 includes a housing 12. Housing 12 can be made from any number of materials including stainless steel, titanium, or polymeric materials. Housing 12 has a distal back portion or closed distal end 14 and a perimeter wall portion 16 extending proximally from the back portion 14 at an angle. Wall portion 16 defines a proximal opening or open proximal end 18, and a fluid reservoir 20 between opening 18 and back portion 14. The port includes a needle penetrable septum 22 attached to the housing about the opening 18 so as to cover the opening and seal the reservoir 20. Septum 22 can be made from any number of materials including silicone. Septum 22 is preferably placed in a proximal enough position such that the depth of the reservoir 20 is sufficient enough to expose the open tip of a needle, such as a Huber needle, so that fluid transfer can take place. Septum 22 is preferably arranged so that it will self seal after being punctured by a needle and the needle is withdrawn. In one embodiment, the septum is made from silicone which is under compression when attached to the housing. Port 10 further includes a catheter tube connection member 30, in fluid communication with reservoir 20.

Port 10 is implanted into a patient and attached to the fascia just below the skin of the patient, so that fluid can be inserted and withdrawn from the inflatable portion with a syringe. As seen from the figures, port 1 includes one or more attachment mechanisms 70, taking the form of an arcuate hook.

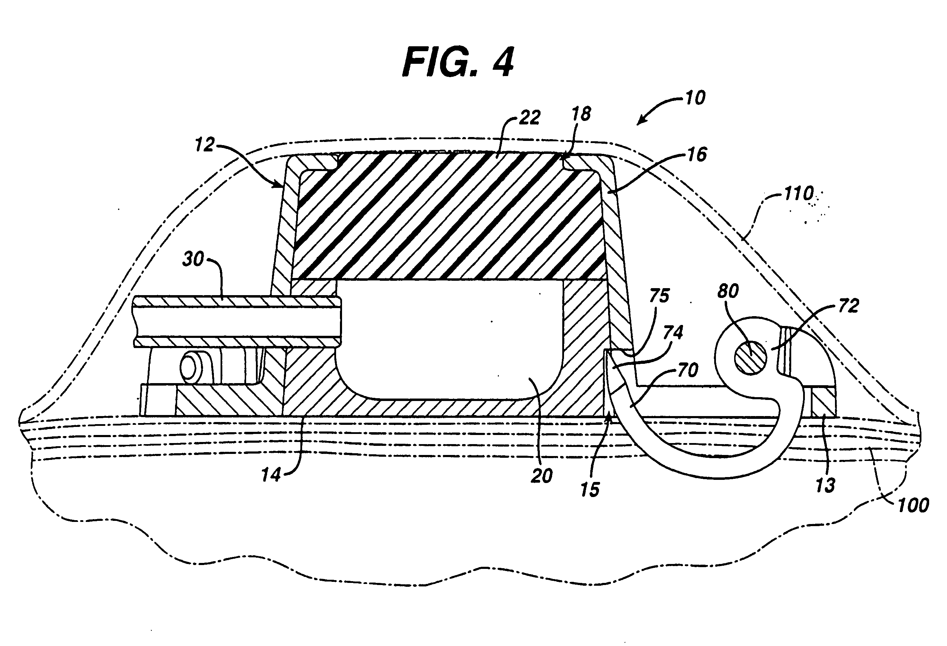

As seen from the figures, port 1 includes one or more attachment mechanisms 70. The figures herein show three attachment mechanisms all substantially identical and equally spaced from each other. Attachment mechanisms 70 are mounted to the housing 12 at a pivot point 80 along an outer periphery 13 of the housing 12. As seen from the figures, attachment mechanisms 70 are arcuate hooks pivotable with respect to the housing. Attachment mechanisms 70 have an arcuate length L extending substantially greater than 90°, and preferably at least 180° about the pivot point. Implantable surgical injection port 10 has an undeployed position, shown as a solid line in Figure 3, and a deployed position, shown as the phantom line in Figure 3 and in Figure 4, wherein the port is attached to tissue. Attachment mechanisms 70 is preferably made from a bioabsorbable material including, but not limited to, one or more of the following either alone or in combination: polydioxanone, polyglactin and/or poliglecaprone.

Attachment mechanism 70 has a fixed end 72 pivotally attached to the housing 12 at pivot point 80. The design allows a surgeon to use forceps and drive the fastener through the tissue until the free end 74 rests against the flat 75. In this way the patient is protected from the sharp end of the tip. Attachment mechanism 70 also includes a free end 74 which has a sharp or pointed configuration. Housing 12 further includes at least one recessed portion 15 along its distal end 14. Recessed portion 15 is designed to receive the free end 74 of attachment mechanisms 70 when the port 1 is in its deployed position. This design prevents any exposure of the sharp free end to tissue after the port has been implanted.

The above described 180° hook or attachment mechanisms provide advantages over prior 90° or less hooks. As seen from Figure 4, the above described attachment mechanism allows the hook to engage a greater area of tissue, and allows for two locking points, entry into and then out of the fascia. This provides for better securement of the port to the tissue. Further no "sharp" is exposed to the patient. A further advantage of the fastener configuration is that the fastener follows a constant radius when pushing through the tissue. By maintaining a constant radius the fastener never induces a compressive force onto the fascia. This should minimize pain because the fastener is not "compressing or squeezing" nerves.

In practice, the physician would create an incision in the skin 110 of a patient to expose the fascia according to well known surgical techniques. Thereafter, as seen from Figure 4, the port 1 could be placed against the fascia 100 of the patient with the port in its undeployed position. Thereafter, the physician could rotate, manually or otherwise, the attachment mechanism substantially greater than 90° and preferably at least 180° so that the hook enters and then exits the fascia. The design allows a surgeon to use forceps and drive the fastener through the tissue until the free end 74 rests against the flat 75. In this way the patient is protected from the sharp end of the tip. This could be done for each attachment mechanism on the device. Thereafter, the catheter tube 52 would be connected to connection member 30, and the patient is sewn up.

It will become readily apparent to those skilled in the art that the above invention has equally applicability to other types of implantable bands. For example, bands are used for the treatment of fecal incontinence. One such band is described in U.S. Patent 6,461,292. Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729.

While preferred embodiments of the present invention have been shown and described herein, it will be obvious to those skilled in the art that such embodiments are provided by way of example only. Numerous variations, changes, and substitutions will now occur to those skilled in the art without departing from the invention as defined into claims. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. An implantable surgical injection port (10) having an undeployed position, and a deployed position wherein it is attached to tissue, said port (10) comprising:
a. a housing (12) having a closed distal end (14), an open proximal end (18) and a fluid reservoir (20) therebetween;
b. a needle penetrable septum (22) attached to said housing (12) about said open proximal end (18); and
c. at least one attachment mechanism taking the form of an arcuate hook, mounted to said housing at a pivot point (80) along an outer periphery of said housing (12), and pivotable with respect to the housing about said pivot point for initially attaching said port to tissue; and
**characterised by:**
said attachment mechanism comprising a bioabsorbable material, and
said arcuate hook having a length extending substantially greater than 90° about said pivot point.

2. The injection port of claim 1 wherein arcuate hook has a length extending substantially at least 180° about said pivot point.

3. The injection port of claim 1 or 2 wherein said attachment mechanism comprises polyglactin.

4. The injection port of claim 1 or 2 wherein said attachment mechanism comprises poliglecaprone.

5. The injection port of any one of preceding claims, wherein said attachment mechanism has a fixed end at the pivot point (80) and a free end at the other end, and the housing has a flat surface against which the free end rests when said attachment mechanism has attached said port to tissue to protect the patient from the sharp end of the tip of the arcuate hook at the free end.

6. The injection port of any one of claims 1 to 5 further including a catheter connection tube attached to said housing and in fluid communication with said reservoir.

7. The injection port of any one of claims 1 to 6 wherein said housing comprises titanium.

8. The injection port of any one of claims 1 to 7 wherein said septum self seals after being punctured by a needle and the needle is withdrawn.

9. The injection port of any one of claims 1 to 8 wherein said septum comprises silicone.

10. The injection port of any one of claims 1 to 9, further including:
d. an adjustable gastric band attached to said housing via a catheter connection tube attached to said housing and in fluid communication with said reservoir.

## Patentansprüche

1. Implantierbarer chirurgischer Injektionsport (10) mit einer nicht eingesetzten Position und einer eingesetzten Position, in der er an Gewebe befestigt ist, wobei der Port (10) aufweist:
a. ein Gehäuse (12) mit einem geschlossenen distalen Ende (14), einem offenen proximalen Ende (18) und einem Fluidbehälter (20) zwischen diesen;
b. ein Septum (22), das von einer Nadel durchdringbar ist und an dem Gehäuse (12) um das offene proximale Ende (18) befestigt ist; und
c. wenigstens einen Befestigungsmechanismus, der die Form eines gebogenen Hakens hat, welcher an dem Gehäuse an einem Schwenkpunkt (80) entlang einem Außenumfang des Gehäuses (12) angebracht ist und welcher in bezug auf das Gehäuse um den Schwenkpunkt verschwenkbar ist, um anfangs den Port am Gewebe zu befestigen; und
**dadurch gekennzeichnet, daß**:
der Befestigungsmechanismus ein bioabsorbierbares Material aufweist und
der gebogene Haken eine Länge hat, die sich im wesentlichen mehr als 90° um den Schwenkpunkt erstreckt.

2. Injektionsport nach Anspruch 1, bei dem der gebogene Haken eine Länge hat, die sich im wesentlichen wenigstens 180° um den Schwenkpunkt erstreckt.

3. Injektionsport nach Anspruch 1 oder 2, bei dem der Befestigungsmechanismus Polyglactin aufweist.

4. Injektionsport nach Anspruch 1 oder 2, bei dem der Befestigungsmechanismus Poliglecapron aufweist.

5. Injektionsport nach einem der vorangehenden Ansprüche, bei dem der Befestigungsmechanismus ein festes Ende an dem Schwenkpunkt (80) und ein freies Ende an dem anderen Ende hat und das Gehäuse eine flache Fläche aufweist, an der das freie Ende ruht, wenn der Befestigungsmechanismus den Port am Gewebe befestigt hat, um den Patienten vor dem scharfen Ende der Spitze des gebogenen Hakens an dem freien Ende zu schützen.

6. Injektionsport nach einem der Ansprüche 1 bis 5, der weiter ein Rohr für die Verbindung mit einem Katheter umfaßt, das an dem Gehäuse befestigt ist und in Fluidverbindung mit dem Behälter steht.

7. Injektionsport nach einem der Ansprüche 1 bis 6, bei dem das Gehäuse Titan aufweist.

8. Injektionsport nach einem der Ansprüche 1 bis 7, bei dem sich das Septum selbst dichtet, nachdem es von einer Nadel durchstochen ist und die Nadel zurückgezogen ist.

9. Injektionsport nach einem der Ansprüche 1 bis 8, bei dem das Septum Silikon aufweist.

10. Injektionsport nach einem der Ansprüche 1 bis 9, der weiter umfaßt:
d. ein einstellbares Magenband, das an dem Gehäuse über ein Rohr für die Verbindung mit einem Katheter befestigt ist, welches an dem Gehäuse befestigt ist und in Fluidverbindung mit dem Behälter steht.

## Revendications

1. Orifice d'injection chirurgical implantable (10) ayant une position non déployée, et une position déployée dans laquelle il est fixé au tissu, ledit orifice (10) comprenant :
a. un boîtier (12) ayant une extrémité distale fermée (14), une extrémité proximale ouverte (18) et un réservoir de fluide (20) entre elles ;
b. un septum (22) pouvant être pénétré par une aiguille fixé audit boîtier (12) autour de ladite extrémité proximale ouverte (18) ; et
c. au moins un mécanisme de fixation prenant la forme d'un crochet arqué, monté sur ledit boîtier au niveau d'un point de pivot (80) le long d'une périphérie externe dudit boîtier (12), et pouvant pivoter par rapport au boîtier autour dudit point de pivot pour fixer initialement ledit orifice au tissu ; et
**caractérisé par** :
◆ ledit mécanisme de fixation comprenant un matériau bioabsorbable, et
◆ ledit crochet arqué ayant une longueur s'étendant sensiblement sur plus de 90° autour dudit point de pivot.

2. Orifice d'injection selon la revendication 1, dans lequel le crochet arqué a une longueur s'étendant sensiblement au moins à 180° autour dudit point de pivot.

3. Orifice d'injection selon la revendication 1 ou 2, dans lequel ledit mécanisme de fixation comprend de la polyglactine.

4. Orifice d'injection selon la revendication 1 ou 2, dans lequel ledit mécanisme de fixation comprend du poliglecaprone.

5. Orifice d'injection selon l'une quelconque des revendications précédentes, dans lequel ledit mécanisme de fixation a une extrémité fixe au niveau du point de pivot (80) et une extrémité libre au niveau de l'autre extrémité, et le boîtier a une surface plate contre laquelle l'extrémité libre repose lorsque ledit mécanisme de fixation a fixé ledit orifice au tissu pour protéger le patient de l'extrémité taillante de la pointe du crochet arqué au niveau de l'extrémité libre.

6. Orifice d'injection selon l'une quelconque des revendications 1 à 5, comprenant en outre un tube de raccordement de cathéter fixé sur ledit boîtier et en communication de fluide avec ledit réservoir.

7. Orifice d'injection selon l'une quelconque des revendications 1 à 6, dans lequel ledit boîtier comprend du titane.

8. Orifice d'injection selon l'une quelconque des revendications 1 à 7, dans lequel ledit septum réalise automatiquement l'étanchéité après avoir été perforé par une aiguille et l'aiguille est retirée.

9. Orifice d'injection selon l'une quelconque des revendications 1 à 8, dans lequel ledit septum comprend de la silicone.

10. Orifice d'injection selon l'une quelconque des revendications 1 à 9, comprenant en outre :
d. une bande gastrique réglable fixée sur ledit boîtier, via un tube de raccordement de cacheter fixé sur ledit boîtier et en communication de fluide avec ledit réservoir.
